# EUROPÄISCHE PATENTSCHRIFT

(11) **EP 1 646 323 B1**
(45) Veröffentlichungstag und Bekanntmachung des Hinweises auf die Patenterteilung: **25.05.2011**
(21) Anmeldenummer: 04763134.6
(22) Anmeldetag: 08.07.2004
(51) Int. Cl.: A61B 18/12, A61B 18/02, A61B 18/18

(54) **CHIRURGISCHE SONDE**
SURGICAL PROBE
SONDE CHIRURGICALE

(30) Priorität: 11.07.2003 DE 10332564
(43) Veröffentlichungstag der Anmeldung: 19.04.2006
(73) Patentinhaber: Celon AG Medical Instruments, 14513 Teltow (DE)
(72) Erfinder: DESINGER, Kai, 12157 Berlin (DE); FAY, Markus, 12249 Berlin (DE); ROGGAN, André, 12163 Berlin (DE)
(74) Vertreter: Eisenführ, Speiser & Partner
(86) Internationale Anmeldenummer: PCT/EP2004/007519
(87) Internationale Veröffentlichungsnummer: WO 2005/006998

(56) Entgegenhaltungen:
- EP-A- 0 856 292
- US-A- 5 281 215
- US-A- 5 913 856
- US-A1- 2002 077 624
- US-A1- 2002 120 258

## Beschreibung

Die Erfindung betrifft eine chirurgische Sonde mit einem Handgriff und einem Schaft, der mit dem Handgriff verbunden ist und wenigstens zwei axial voneinander beabstandete Elektroden aufweist. Eine, dem Handgriff nähere Elektrode bildet eine proximale Elektrode und die andere, dem Handgriff fernere Elektrode bildet eine distale Elektrode. Die Elektroden bilden jeweils eine elektrisch leitende Außenoberfläche des Schaftes und sind durch einen Isolator axial voneinander getrennt. Der Außendurchmesser der beiden Elektroden und der Außendurchmesser des Isolators sind annähernd gleich. Der Schaft weist außerdem eine Fluidpassage auf, welche sich im Inneren des Schaftes vom Handgriff bis in die distale Elektrode erstreckt, so dass proximale und distale Elektrode durch ein Fluid zu temperieren, z.B. zu kühlen oder vorzuwärmen sind. Dem Hauptanwendungsgebiet entsprechend wird im folgenden regelmäßig von Kühlfluid oder -flüssigkeit gesprochen. Hiermit ist in besonderen Anwendungsfällen auch ein Fluid zum Temperieren oder auch Erwärmen der Sonde gemeint.

Derartige chirurgische Sonden sind grundsätzlich bekannt und dienen beispielsweise der Gewebeablation oder Koagulation. Zu diesem Zweck wird der Schaft mit beiden Elektroden in Körpergewebe eingestochen. An beide Elektroden wird beispielsweise eine hochfrequente Wechselspannung angelegt, die einen Wechselstrom in dem den Schaft umgebenden Körpergewebe verursacht und dessen Erwärmung bewirkt. Der Hochfrequenzstrom und die Elektrodengeometrie sind dabei so gewählt, dass die in Folge des Hochfrequenzstroms eintretende Erwärmung des Körpergewebes zum Zelltod und damit zur Gewebeverödung führt. Auf diese Weise lassen sich beispielsweise Tumore behandeln.

Um die in das Körpergewebe eingetragene Energie besser zu verteilen und zu verhindern, dass beispielsweise Köpergewebe in der Nähe der Elektroden nahezu verbrennt, während bereits in geringem Abstand von den Elektroden kaum noch Wärme erzeugt wird, ist es bekannt, die Elektroden während der Abgabe des Hochfrequenzstromes zu kühlen oder zur Ablation des Punktionskanals zu erwärmen. Da die Kühlung dort besonders wirksam ist, wo die elektrischen Feldstärken besonders hoch sind, kann auf diese Weise die Erwärmung des Körpergewebes gleichmäßiger und auf ein größeres Volumen verteilt werden. Dokument US 2002/077624 offenbart ein gerät gemäß dem oberbegriff des Anspruchs 1.

Der Wunsch, die Elektroden temperieren zu können, bringt für den Aufbau des Schaftes der chirurgische Sonde weitere Probleme mit sich. Zum einen kann die Kühlflüssigkeit leitend sein (beispielsweise physiologische Kochsalzlösung), so dass die Kühlflüssigkeit zu Kurzschlüssen zwischen den beiden Elektroden im Inneren des Schaftes führen kann. Zum anderen erfordert die Fluidpassage für das Kühlfluid einen hohlen Elektrodenaufbau, so dass bei gleichzeitig gewünschtem geringen Durchmesser des Schaftes nur schwer alle Anforderungen an die mechanische Festigkeit und Dichtigkeit des Schaftes erfüllt werden können. Dies gilt umso mehr, als der Schaft der chirurgischen Sonde für die Behandlung von Körpergewebe üblicherweise in dieses eingestochen wird.

Die bekannten Ansätzen, alle Anforderungen an eine fluidtemperierte chirurgische Sonde für eine interstitielle Thermotherapie gleichermaßen gut zu erfüllen, sind zumeist nicht völlig befriedigend. Der Erfindung liegt daher die Aufgabe zu Grunde, eine chirurgische Sonde anzugeben, die gute Kühleigenschaften besitzt, gute elektrische Eigenschaften besitzt, eine hohe mechanische Festigkeit besitzt und außerdem bei hohen Fluiddrücken dicht ist.

Erfindungsgemäß wird diese Aufgabe durch eine chirurgische Sonde der Eingangs genannten Art gelöst, bei der der Schaft einen distal verschlossenen Hohlkörper aufweist, der mit dem Handgriff verbunden ist, die distale Elektrode bildet und den Isolator sowie die proximale Elektrode trägt. Außerdem weist dieser Schaft eine Isolationsschicht auf, die in radialer Richtung zwischen dem Hohlkörper und der proximalen Elektrode angeordnet ist.

Der Hohlkörper ist vorzugsweise einstückig ausgeführt. Ein besonders bevorzugter, einstückiger Hohlköper ist durch Verschweißen eines ersten rohrförmigen Bauteils mit einem das verschlossene distale Ende bildenden zweiten Bauteil hergestellt.

Es hat sich nämlich gezeigt, dass es möglich ist, eine grundsätzlich die Kühlung der proximalen Elektrode beeinträchtigende elektrische Isolationsschicht so auszuführen, dass die Wärmeleitung durch die Isolationsschicht praktisch nicht beeinträchtigt wird.

Um die Kühlung der proximalen Elektrode möglichst wenig zu beeinträchtigen, ist die Isolationsschicht vorzugsweise nur wenige Mikrometer dick, beispielsweise zwischen 1 und 10 µm.

Die Isolationsschicht ist vorzugsweise sowohl zwischen dem Hohlkörper und der proximalen Elektrode als auch zwischen dem Hohlkörper und dem Isolator angeordnet. In einer insbesondere auch unter Fertigungsgesichtspunkten besonders bevorzugten Ausführungsvariante ist die Isolierschicht von Schrumpfschlauch gebildet.

Der Isolator und vorzugsweise auch die proximale Elektrode sind vorzugsweise jeweils als Rohr mit im wesentlichen einheitlicher Wandstärke ausgebildet, so dass der Isolator und die proximale Elektrode auf den Hohlkörper und den auf den Hohlkörper aufgeschrumpften Schrumpfschlauch aufzuschieben sind.

Der Hohlkörper besitzt vorzugsweise einen Abschnitt mit einem vergrößerten Außendurchmesser dort, wo der Hohlkörper die distale Elektrode bildet. Proximal von diesem Abschnitt weist der Hohlkörper vorzugsweise einen geringeren Durchmesser auf. Auf diesen Abschnitt des Hohlkörpers mit geringerem Durchmesser kann der Schrumpfschlauch aufgeschoben und aufgeschrumpft werden. Anschließend können auf diesen Abschnitt der Isolator und die proximale Elektrode aufgeschoben werden. Die Durchmesser von Hohlkörper, Isolator und proximaler Elektrode sind vorzugsweise so bemessen, dass der Innendurchmesser von Isolator und proximaler Elektrode ein Aufschieben auf den Schrumpfschlauch erlaubt. Außerdem sollen der Außendurchmesser des die distale Elektrode bildenden Abschnittes des Hohlkörpers mit vergrößertem Durchmesser sowie die Außendurchmesser von Isolator und proximaler Elektrode einander möglichst gleich sein, so dass sich ein Schaft mit möglichst durchgängig gleichem Außendurchmesser ergibt. Annähernd gleich bedeutet in diesem Falle, dass sich die Außendurchmesser der distalen Elektrode, des Isolators und proximalen Elektroden dem Rahmen der Fertigungsgenauigkeiten entsprechen sollen. Für eine derartige Ausführungsform ist die proximale Elektrode vorzugsweise als Metallrohr mit über seine Länge im wesentlichen gleichen Außen- und Innendurchmesser, d.h. auch im wesentlichen gleicher Wandstärke ausgebildet. Die Formulierung "im wesentlichen gleich" schließt Anfasungen und Gewinde an der proximalen Elektrode ebenso wenig aus wie Fertigungsungenauigkeiten.

In einer alternativen Ausführungsvariante nimmt der Außendurchmesser der proximalen Elektrode ausgehend vom Handgriff in Richtung des distalen Endes des Schaftes kontinuierlich ab, so dass sich eine beispielsweise konisch geformte proximale Elektrode ergibt.

Der Hohlkörper ist vorzugsweise an seinem distalen Ende verschlossen, so dass im Bereich des Schaftes kein Fluid aus der Fluidpassage austreten kann. Die Fluidpassage im Inneren des Hohlkörpers erstreckt sich vorzugsweise bis zu dem verschlossenen Ende des Hohlkörpers und besitzt in einer besonders bevorzugten Ausführungsvariante einen durchgängig gleichen Durchmesser. Auf diese Weise lässt sich die Fluidpassage leicht fertigen.

Die chirurgische Sonde weist außerdem vorzugsweise einen Schlauch im Inneren der Fluidpassage auf, der eine Mündung in der Nähe des verschlossenen Endes der Fluidpassage besitzt und so angeordnet und angeschlossen ist, dass ein Kühlfluid durch den Schlauch bis in die Nähe des distalen Endes der Fluidpassage zu leiten ist, dort aus der Mündung des Schlauches austritt und zwischen Schlauch und Wand der Fluidpassage zum proximalen Ende des Schaftes zurückströmen kann.

Hierzu besitzt der Schlauch einen Außendurchmesser, der kleiner ist, als der Innendurchmesser der Fluidpassage. Auf diese Weise lässt sich eine Zuführung für das Kühlfluid bis zum distalen Ende der Fluidpassage leicht durch Einschieben eines entsprechenden Schlauches herstellen.

Der Schaft der chirurgischen Sonde ist an seinem distalen Ende auf der Außenseite vorzugsweise angespitzt, um eine leichtes Einstechen des Schaftes in Körpergewebe zu ermöglichen.

An seinem proximalen Ende ist der Schaft mit dem Handgriff verbunden und dort vorzugsweise teilweise in Dichtmasse so eingebettet, dass das die proximale Elektrode bildende Rohr an dessen proximalem Ende vollständig in die Dichtmasse eingebettet ist, während das proximale Ende des Hohlkörpers aus der Dichtmasse herausragt. Dabei ist die proximale Elektrode vorzugsweise innerhalb der Dichtmasse elektrisch kontaktiert, d.h. mit einer elektrischen Leitung verbunden, mittels der sich die proximale Elektrode an einen Hochfrequenzgenerator anschließen lässt. Mit Hilfe der Dichtmasse lassen sich wirkungsvoll Kurzschlüsse zwischen proximaler Elektrode und Hohlkörper selbst dann verhindern, wenn über den Handgriff der chirurgischen Sonde ein elektrisch leitendes Kühlfluid in den Schaft eingespült wird.

Das proximale Ende des Hohlkörpers ragt vorzugsweise über das proximale Ende der proximalen Elektrode hinaus und somit weiter in den Handgriff hinein, so dass der Hohlkörper und damit auch die distale Elektrode in der Nähe des proximalen Endes des Hohlkörpers elektrisch kontaktiert werden kann.

Die Erfindung soll nun anhand eines in den Figuren abgebildeten Ausführungsbeispiels näher erläutert werden.

Von den Figuren zeigen:
- Figur 1: eine erfindungsgemäße chirurgische Sonde mit Handgriff und Schaft;
- Figur 2: die chirurgische Sonde aus Fig. 1 in einer längsgeschnittenen Darstellung;
- Figur 3: das distale Ende des Schaftes der chirurgischen Sonde aus Fig. 1 und 2 in längsgeschnittener, vergrößerter Darstellung;
- Figur 4: ein nochmals vergrößerter Ausschnitt aus Fig. 3; und
- Figur 5: das proximale Ende der chirurgischen Sonde aus Fig. 1 und 2 in vergrößerter, längsgeschnittener Darstellung.

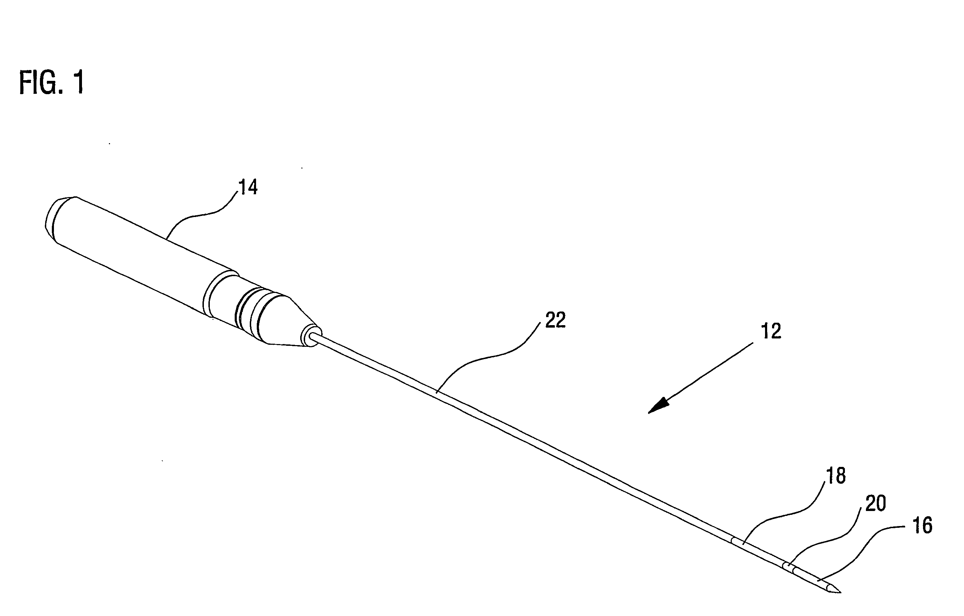

Die in Fig. 1 abgebildete chirurgische Sonde 10 besitzt einen längsgestreckten Schaft 12, der in seinem proximalen Ende mit einem Handgriff 14 verbunden ist. Am distalen Ende des Schaftes 12 sind zwei Elektroden 16 und 18 vorgesehen, nämlich eine distale Spitzenelektrode 16 und eine proximale Elektrode 18. Zwischen beiden Elektroden ist ein Isolator 20 angeordnet. Die Elektroden 16 und 18 sowie der Isolator 20 haben annähernd den gleichen Außendurchmesser. In proximaler Richtung ist außerdem eine äußere Isolierschicht 22 vorgesehen, die bewirkt, dass die wirksame Elektrodenfläche der proximalen Elektrode 18 ähnlich groß ist, wie die wirksame Elektrodenfläche der distalen Elektrode 16.

In einer bevorzugten Ausführungsvariante ist die proximale Elektrodenfläche etwa 10% größer als die distale Elektrodenfläche, um Kühlbeeinträchtigungen der proximalen Elektrode 18 infolge der Isolierschicht zu kompensieren.

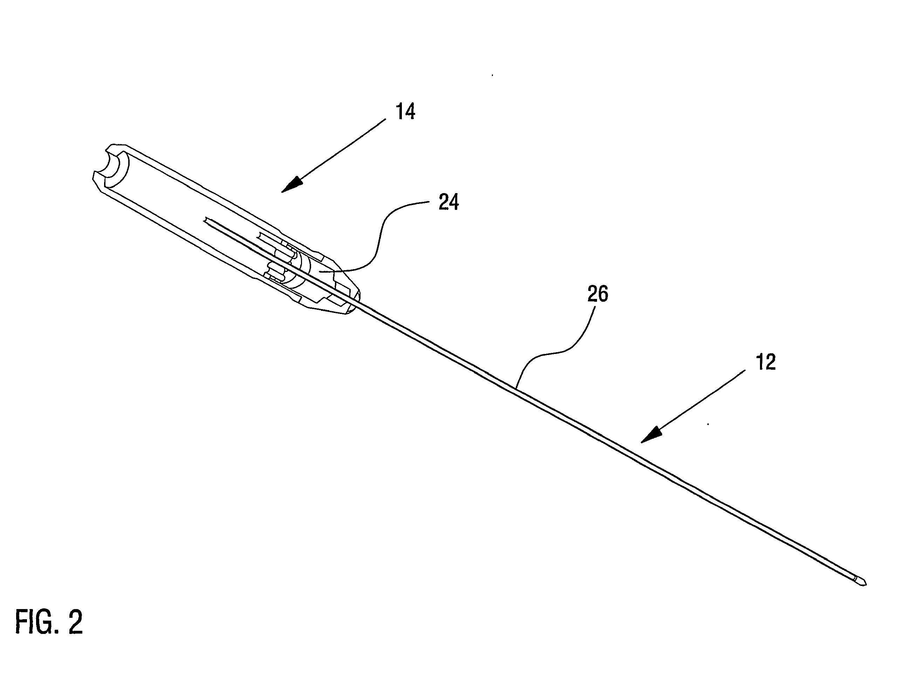

Fig. 2 zeigt die chirurgische Sonde 10 aus Fig. 1 im Längsschnitt. Zu erkennen ist, dass der Handgriff 14 einen Hohlraum 24 aufweist und außerdem ist der Schaft 12 über den größten Teil seiner Länge im inneren hohl. Der Hohlraum bildet eine Fluidpassage 26, die mit dem Hohlraum 24 in Fluidverbindung steht.

Der vergrößerten, längsgeschnittenen Darstellung des distalen Endes des Schaftes 12 ist der Aufbau des Schaftes 12 im Detail zu entnehmen: Bestandteile des Schaftes 12 sind ein längsgestreckter Hohlkörper 30 mit einem als Fluidpassage 26 an sich dienenden Hohlraum in seinem Inneren, einem distalen Ende 32 mit vergrößertem Außendurchmesser und einem proximalen Abschnitt 34 mit geringerem Außendurchmesser; sowie eine innere Isolierschicht 36, sowie ein Isolator 38 und ein unter anderem die proximale Elektrode bildendes Metallrohr 40. An seinem distalen Ende trägt das Metallrohr 40 die äußere Isolierschicht 22.

Die äußere Oberfläche des distalen Abschnitts 32 des Hohlkörpers 30 bildet die distale Spitzenelektrode 16.

Die innere Isolierschicht 36 auf dem proximalen Abschnitt 34 ist von einem Schrumpfschlauch gebildet, der außen auf den proximalen Abschnitt 34 aufgeschoben ist und eine Wandstärke von etwa 5 bis 10 µm besitzt. Mit dieser Wandstärke ist einerseits eine gute elektrische Durchschlagsicherheit gegeben, andererseits eine immer noch gute Wärmeleitung zwischen proximalem Abschnitt 34 des Hohlkörpers 30 und dem Metallrohr 40.

Außen auf dem Schrumpfschlauch 36 sind der Isolator 38 und das Metallrohr 40 so aufgeschoben, dass der Isolator 38 in Längsrichtung zwischen dem distalen Abschnitt 32 des Hohlkörpers 30 und dem Metallrohr 40 angeordnet ist. Der Isolator 38 besteht beispielsweise aus einem dünnen, thermostabilen Kunststoffröhrchen, beispielsweise aus PTFE oder PEEK.

Die äußere Isolierschicht 22, die außen auf dem Metallrohr 40 aufgebracht ist, ist ebenfalls von Schrumpfschlauch gebildet. Die zwischen dem Isolator 38 und der äußeren Isolierschicht 22 blank liegende Außenoberfläche des Metallrohres 40 bildet die proximale Elektrode 18.

Eine elektrische Verbindung der distalen Elektrode 16 mit einem elektrischen Anschluss im Handgriff 14 ist über den proximalen Abschnitt 34 des Hohlkörpers 30 gegeben. Der Hohlkörper 30 besteht aus Metall.

Die proximale Elektrode 18 ist über das Metallrohr 40 mit einem elektrischen Anschluss im Inneren des Handgriffs 14 elektrisch verbunden. In der Fluidpassage 26 im Inneren des Hohlkörpers 30 ist ein Kunststoffschlauch 42 angeordnet, durch den hindurch ein Kühlfluid bis in die Nähe des distalen Endes der Fluidpassage 26 gespült werden kann. Das Kühlfluid strömt dann auf der Außenseite des Kunststoffschlauches 42 zwischen Kunststoffschlauch 42 und Innenwand der Fluidpassage 26 im Inneren des Hohlkörpers 30 zurück zum Handgriff 14.

Die Fluidpassage 26 ist von einer Bohrung im Inneren des Hohlkörpers 30 gebildet, die aus fertigungstechnischen Gründen vorzugsweise durchgehend den gleichen Durchmesser aufweisen.

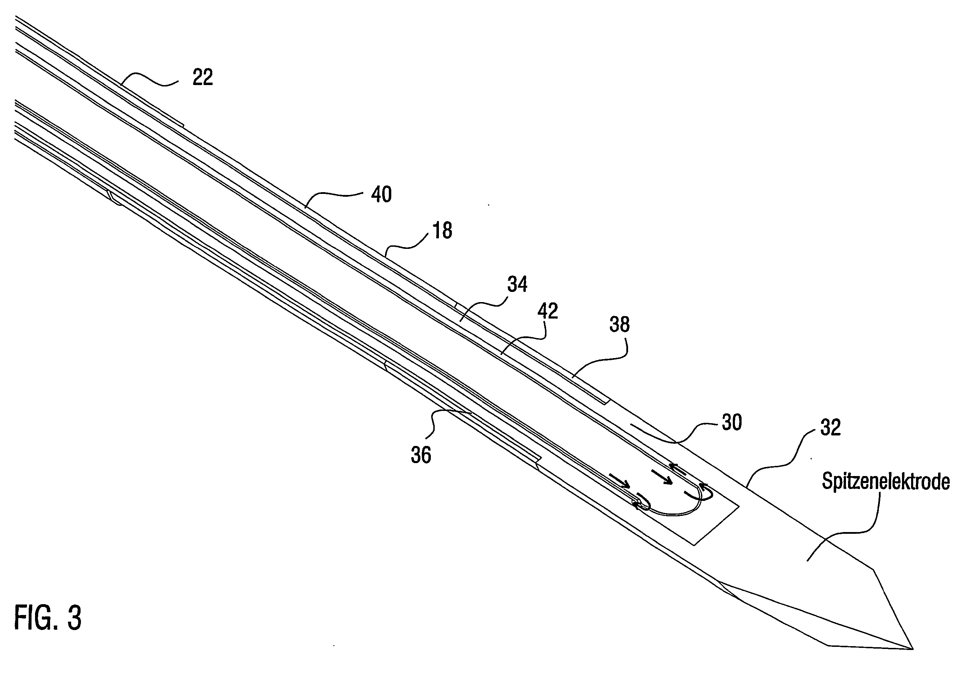

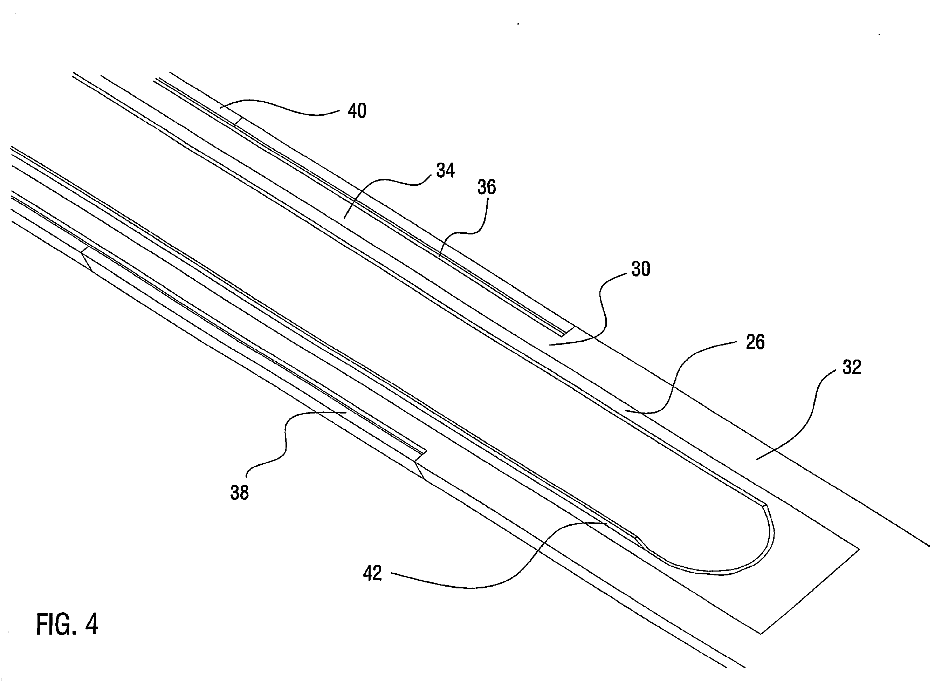

In dem in Fig. 4 dargestellten, vergrößerten Ausschnitt des Längsschnitts aus Fig. 3 ist die relative Anordnung des Hohlkörpers 30 mit distalem Abschnitt 32 und proximalem Abschnitt 34 sowie in den liegender Fluidpassagen 36 relativ zum Kunststoffschlauch 42 einerseits sowie zur inneren Isolierschicht 36, dem Isolator 38 und dem Metallrohr 40 noch genauer zu erkennen.

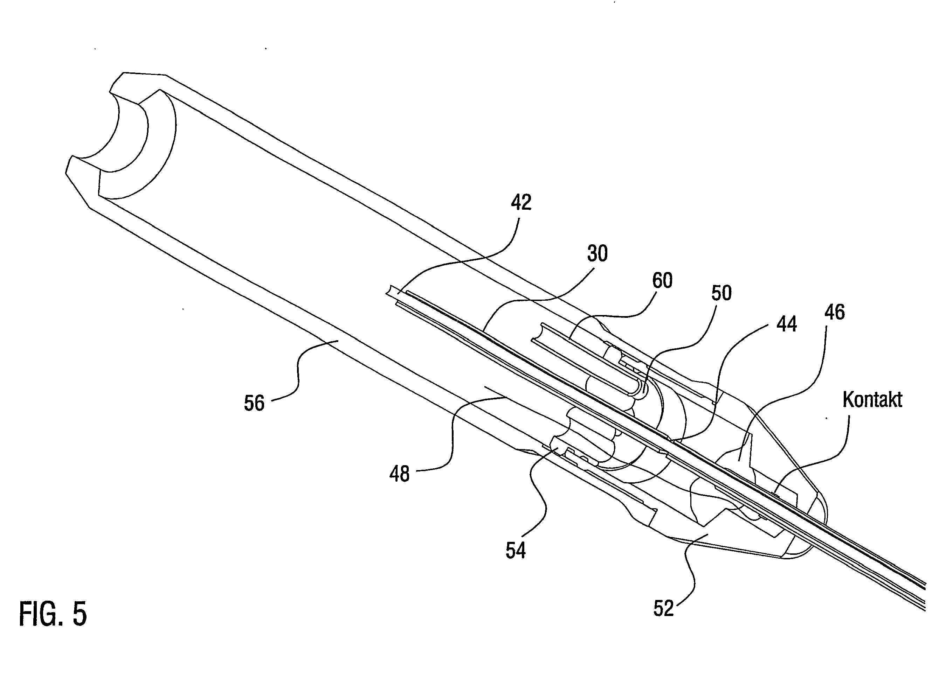

Die gegenüber Fig. 2 vergrößerte, längsgeschnittene Darstellung in Fig. 5 zeigt den Handgriff 14 im Längsschnitt sowie das proximale Ende des Schaftes 12.

Bezüglich des Schaftes 12 ist bemerkenswert, dass sich das Metallrohr 40 proximal weiter in Handgriff 14 hineinerstreckt, als die äußere Isolierschicht. Weiterhin erstreckt sich die innere Isolierschicht 36 weiter ins Innere des Handgriffs 14, als das Metallrohr 40. Der Hohlkörper 30 erstreckt sich sogar erheblich weiter ins Innere des Handgriffs 14, als die innere Isolierschicht 36 und weist jenseits des proximalen Endes der inneren Isolierschicht 36 eine Querbohrung 44 auf, durch die Flüssigkeit aus dem die Fluidpassage 26 bildenden Hohlraum im inneren des Hohlkörpers 30 nach außen austreten kann.

Schließlich ragt der Kunststoffschlauch 42 proximal aus der Fluidpassage 26 heraus, so dass der Kunststoffschlauch 42 beispielsweise mit einer Pumpe für ein Kühlfluid verbunden werden kann. Das Kühlfluid wird durch die Pumpe am besten bis zum distalen Ende des Kunststoffschlauchs 42 gefördert, tritt dort aus einer Mündung des Kunststoffschlauchs 42 aus und strömt zwischen Außenwand des Kunststoffschlauchs 42 und Innenwand der Fluidpassage 36 zurück in den Handgriff 40, um dort über die Querbohrung 44 aus der Fluidpassage 26 auszutreten.

Dort, wo die äußere Isolierschicht 22 und das Metallrohr 40 proximal enden, ist der Schaft 12 von einer elektrisch isolierenden Dichtmasse 46 umgeben. Innerhalb der isolierenden Dichtmasse 46 ist das proximale Ende des Metallrohrs 40 elektrisch kontaktiert, so dass es mittels eines Kabels 48 mit einem Pol eines Generators für eine Hochfrequenzspannung zu verbinden ist.

Das proximale Ende des Hohlkörpers 30 ist mit einem zweiten Kabel 50 elektrisch verbunden, mittels welchem eine elektrische Verbindung zu einem zweiten Pol eines Generators für Hochfrequenzspannung zu verbinden ist.

Bezüglich des Aufbaus des Handgriffs 14 ist Fig. 5 zu entnehmen, dass ein distales Endstück 52 des Handgriffs 14 eine erste Unterhalterung für den Schaft 12 bildet. In einer Öffnung am proximalen Ende des distalen Endes 52 ist ein proximales Haltestück 54 eingeschoben, welches eine zweite Halterung für den Schaft 12 bildet. Die Einheit aus distalem Endstück 52 und proximaler Halterung 54 ist in eine distale Öffnung eines Handstücks 56 eingeschoben.

Das distale Endstück 52 und die proximale Halterung 54 schließen den Hohlraum 24 ein. In diesem Hohlraum 24 ist auch die Querbohrung 44 in dem Hohlkörper 30 angeordnet, so dass aus der Fluidpassage 26 austretendes Kühlfluid in den Hohlraum 26 strömt und über eine in eine Durchgangsbohrung 58 in der proximalen Halterung 54 eingesteckte Leitung 60 abfließen kann. Eine zweite Durchgangsbohrung 62 in der proximalen Halterung 54 dienst als Durchführung für das Kabel 48 und ist im übrigen mit Dichtmasse abgedichtet.

## Patentansprüche

1. Chirurgische Sonde (10) mit einem Handgriff (14) und einem Schaft (12), der mit dem Handgriff (14) verbunden ist und zwei axial voneinander beabstandete Elektroden (16, 18) aufweist, von denen eine dem Handgriff nähere Elektrode eine proximale Elektrode (18) bildet und die andere, dem Handgriff ferne Elektrode eine distale Elektrode (16) bildet, wobei die Elektroden jeweils eine Auβenoberfläche des Schafts bilden und durch einen Isolator (20) voneinander getrennt sind, wobei der Außendurchmesser der beiden Elektroden (16, 18) und der Außerdurchmesser des Isolators (20) annähernd gleich sind und wobei der Schaft (12) ein Fluidpassage (26) für ein Kühlfluid aufweist, welche sich im Inneren des Schaftes vom Handgriff bis in die distale Elektrode erstreckt, **dadurch gekennzeichnet, dass** der Schaft einen distal verschlossenen Hohlkörper (30) aufweist, der mit dem Handgriff (14) verbunden ist und die distale Elektrode (16) bildet, den Isolator (20) sowie die proximale Elektrode (18) und eine Isolierschicht (36) trägt, die in radialer Richtung zwischen dem
Hohlkörper (30) und der proximalen Elektrode (18) angeordnet ist.

2. Chirurgische Sonde nach Anspruch 1, **dadurch gekennzeichnet, dass** die Isolierschicht (36) sowohl zwischen Hohlkörper (30) und proximaler Elektrode (18) als auch zwischen Hohlkörper (30) und Isolator (20) angeordnet ist.

3. Chirurgische Sonde nach Anspruch 1 oder 2, **dadurch gekennzeichnet, dass** die Isolierschicht (36) von Schrumpfschlauch gebildet ist.

4. Chirurgische Sonde nach einem der Ansprüche 1 bis 3, **dadurch gekennzeichnet, dass** die proximale Elektrode (18) von einem Metallrohr (40) mit einem über seine Länge im wesentlichen gleichen Durchmesser und im wesentlichen gleicher Wandstärke gebildet ist.

5. Chirurgische Sonde nach einem der Ansprüche 1 bis 4, **dadurch gekennzeichnet, dass** der Hohlkörper (30) an seinem distalen Ende verschlossen ist.

6. Chirurgische Sonde nach Anspruch 5, **dadurch gekennzeichnet, dass** sich die Fluidpassage (26) in dem Hohlkörper bis zu dessen verschlossenem Ende erstreckt und einen im wesentlichen durchgängig gleichen Durchmesser besitzt.

7. Chirurgische Sonde nach einem der Ansprüche 1 bis 6, **dadurch gekennzeichnet, dass** der Hohlkörper (30) an seinem distalen Ende angespitzt ist.

8. Chirurgische Sonde nach einem der Ansprüche 1 bis 7, **dadurch gekennzeichnet, dass** der Hohlkörper (30) im Bereich der distalen Elektrode (16) einen Außendurchmesser aufweist, der annähernd gleich dem Außendurchmesser der proximalen Elektrode (18) oder des Isolators (20) ist.

9. Chirurgische Sonde nach einem der Ansprüche 1 bis 8, **dadurch gekennzeichnet, dass** der Hohlkörper (30) im Bereich des Isolators (20) und der proximalen Elektrode (18) einen geringeren Durchmesser aufweist, als im Bereich der distalen Elektrode (16).

10. Chirurgische Sonde nach einem der Ansprüche 1 bis 9, **gekennzeichnet durch** einen Schlauch (42) im Inneren der Fluidpassage (26) mit einer Mündung in der Nähe des verschlossenen distalen Endes der Fluidpassage (26), der so angeordnet ist und angeschlossen ist, dass ein Kühlfluid **durch** den Schlauch (42) bis in die Nähe des distalen Endes der Fluidpassage (26) zu leiten ist, dort aus der Mündung des Schlauches (42) austritt und zwischen Schlauch (42) und Wand der Fluidpassage (26) zum proximalen Ende des Schaftes (12) zurückströmen kann.

11. Chirurgische Sonde nach einem der Ansprüche 1 bis 10, **dadurch gekennzeichnet, dass** der Schaft (12) an seinem proximalen Ende mit dem Handgriff (14) verbunden und dort teilweise in Dichtmasse (46) so eingebettet ist, dass das die proximale Elektrode bildende Rohr (40) an dessen proximalem Ende vollständig in die Dichtmasse (46) eingebettet ist, während das proximale Ende des Hohlköpers (30) aus der Dichtmasse (46) herausragt.

12. Chirurgische Sonde nach Anspruch 11, **dadurch gekennzeichnet, dass** die proximale Elektrode (18) innerhalb der Dichtmasse (46) elektrisch kontaktiert ist.

## Claims

1. Surgical probe (10) comprising a handle (14) and a shaft (12), which is connected to the handle (14) and has two axially mutually spaced electrodes (16, 18), one of the electrodes nearer the handle forming a proximal electrode (18) and the other electrode remote from the handle forming a distal electrode (16), wherein the electrodes respectively form an external surface of the shaft and are separated from one another by an insulator (20), wherein the external diameter of the two electrodes (16, 18) and the external diameter of the insulator (20) are approximately identical and wherein the shaft (12) has a fluid passage (26) for a cooling fluid, which extends inside the shaft from the handle into the distal electrode, **characterised in that** the shaft has a distally closed hollow body (30), which is connected to the handle (14) and forms the distal electrode (16), supports the insulator (20) as well as the proximal electrode (18) and an insulating layer (36) which is arranged in radial direction between the hollow body (30) and the proximal electrode (18).

2. Surgical probe according to claim 1, **characterised in that** the insulating layer (36) is arranged both between the hollow body (30) and the proximal electrode (18) and also between the hollow body (30) and the insulator (20).

3. Surgical probe according to claim 1 or 2, **characterised in that** the insulating layer (36) is formed by shrink tube.

4. Surgical probe according to one of claims 1 to 3, **characterised in that** the proximal electrode (18) is formed by a metal tube (40) with a diameter which is substantially uniform over its length and with a substantially uniform wall thickness.

5. Surgical probe according to one of claims 1 to 4, **characterised in that** the hollow body (30) is closed at its distal end.

6. Surgical probe according to claim 5, **characterised in that** the fluid passage (26) extends in the hollow body up to the closed end thereof and is of a diameter which is substantially uniform throughout.

7. Surgical probe according to one of claims 1 to 6, **characterised in that** the hollow body (30) is pointed at its distal end.

8. Surgical probe according to one of claims 1 to 7, **characterised in that** in the region of the distal electrode (16) the hollow body (30) has an external diameter, which is approximately the same as the external diameter of the proximal electrode (18) or the insulator (20).

9. Surgical probe according to one of claims 1 to 8, **characterised in that** in the region of the insulator (20) and the proximal electrode (18) the hollow body (30) has a smaller diameter than in the region of the distal electrode (16).

10. Surgical probe according to one of claims 1 to 9, **characterised by** a hose (42) in the interior of the fluid passage (26) with an opening in the proximity of the closed distal end of the fluid passage (26), which hose is arranged and connected so that a cooling fluid is directed through the hose (42) into the proximity of the distal end of the fluid passage (26), emerges there out of the opening of the hose (42) and can flow back between the hose (42) and the wall of the fluid passage (26) to the proximal end of the shaft (12).

11. Surgical probe according to one of claims 1 to 10, **characterised in that** at its proximal end the shaft (12) is connected to the handle (14) and is partially embedded there in sealing material (46) in such a way that the tube (40) forming the proximal electrode is completely embedded at its proximal end in the sealing material (46), whilst the proximal end of the hollow body (30) projects out of the sealing material (46).

12. Surgical probe according to claim 11, **characterised in that** the proximal electrode (18) is electrically contacted within the sealing material (46).

## Revendications

1. Sonde chirurgicale (10) comportant une poignée (14) et une tige (12) connectée à la poignée (14), présentant deux électrodes axialement espacées l'une par rapport à l'autre (16, 18), dont l'une électrode proche de la poignée forme une électrode proximale (18) et l'autre électrode, éloignée de la poignée, forme une électrode distale (16), lesdites électrodes formant une surface extérieure de la tige et étant séparées l'une de l'autre par un isolateur (20), le diamètre extérieur des deux électrodes (16, 18) et le diamètre de l'isolateur (20) étant environ égaux et la tige (12) présentant un passage fluidique (26) destiné à du fluide de refroidissement, s'étendant à l'intérieur de la tige, de la poignée à l'électrode distale, **caractérisée en ce que** la tige comporte un corps creux distal fermé (30) connecté à la poignée (14), formant l'électrode distale (16), portant l'isolateur (20), l'électrode proximale (18) et une couche d'isolation (36) disposée dans la direction radiale entre le corps creux (30) et l'électrode proximale (18).

2. Sonde chirurgicale selon la revendication 1 **caractérisée en ce que** la couche d'isolation (36) est disposée aussi bien entre le corps creux (30) et l'électrode proximale (18) qu'entre le corps creux (30) et l'isolateur (20).

3. Sonde chirurgicale selon la revendication 1 ou la revendication 2 **caractérisée en ce que** la couche d'isolation (36) est formée d'une gaine thermorétractable.

4. Sonde chirurgicale selon l'une des revendications 1 à 3 **caractérisée en ce que** l'électrode proximale (18) est formée d'un tube métallique (40) d'un diamètre sensiblement identique sur sa longueur et d'une épaisseur de paroi sensiblement identique.

5. Sonde chirurgicale selon l'une des revendications 1 à 4 **caractérisée en ce que** le corps creux (30) est fermé à son extrémité distale.

6. Sonde chirurgicale selon la revendication 5 **caractérisée en ce que** le passage fluidique (26) s'étend dans le corps creux jusqu'à l'extrémité fermée et possède un diamètre sensiblement identique sur toute sa longueur.

7. Sonde chirurgicale selon l'une des revendications 1 à 6 **caractérisée en ce que** le corps creux (30) est pointu à son extrémité distale.

8. Sonde chirurgicale selon l'une des revendications 1 à 7 **caractérisée en ce que** le corps creux (30) présente un diamètre extérieur au niveau de l'électrode distale (16) environ égal au diamètre extérieur de l'électrode proximale (18) ou de l'isolateur (20).

9. Sonde chirurgicale selon l'une des revendications 1 à 8 **caractérisée en ce que** le corps creux (30) au niveau de l'isolateur (20) et de l'électrode proximale (18) présente un diamètre moins important qu'au niveau de l'électrode distale (16).

10. Sonde chirurgicale selon l'une des revendications 1 à 9 **caractérisée par** un tuyau (42) à l'intérieur du passage fluidique (26) avec un orifice à proximité de l'extrémité distale fermée du passage fluidique (26), tuyau qui est disposé et raccordé de sorte qu'un fluide de refroidissement doit être dirigé à travers le tuyau (42) jusqu'à proximité de l'extrémité distale fermée du passage fluidique (26), qu'à cet endroit il sort par l'orifice du tuyau (42) et qu'il peut refluer entre le tuyau (42) et la paroi du passage fluidique (26) jusqu'à l'extrémité proximale de la tige (12).

11. Sonde chirurgicale selon l'une des revendications 1 à 10 **caractérisée en ce que** la tige (12) est connectée à son extrémité proximale à la poignée (14) et, à cet endroit, est en partie incorporée dans un matériau d'étanchéité (46) de telle sorte que le tube formant l'électrode proximale (40) est entièrement incorporé à son extrémité proximale dans le matériau d'étanchéité (46), tandis que l'extrémité proximale du corps creux (30) fait saillie hors du matériau d'étanchéité (46).

12. Sonde chirurgicale selon la revendication 11 **caractérisée en ce que** l'électrode proximale (18) est mise en contact électrique à l'intérieur du matériau d'étanchéité (46).
